# EUROPÄISCHE PATENTSCHRIFT

(11) **EP 1 781 119 B1**
(45) Veröffentlichungstag und Bekanntmachung des Hinweises auf die Patenterteilung: **27.01.2010**
(21) Anmeldenummer: 05776037.3
(22) Anmeldetag: 18.08.2005
(51) Int. Cl.: A23L 1/30, A61K 31/00

(54) **LIPONSÄURE-KONZENTRAT**
LIPOIC ACID CONCENTRATE
CONCENTRE D'ACIDE LIPOIQUE

(30) Priorität: 18.08.2004 DE 102004040178
(43) Veröffentlichungstag der Anmeldung: 09.05.2007
(73) Patentinhaber: Aquanova AG, 64295 Darmstadt (DE)
(72) Erfinder: BEHNAM, Dariush, 64380 Rossdorf (DE)
(74) Vertreter: Blumbach - Zinngrebe
(86) Internationale Anmeldenummer: PCT/EP2005/008940
(87) Internationale Veröffentlichungsnummer: WO 2006/018301

(56) Entgegenhaltungen:
- WO-A-03/007907
- US-B1- 6 300 377

## Beschreibung

Die Erfindung betrifft ein α-Liponsäure-Konzentrat gemäß DE 101 08 614 A1.

Seit der Untersuchung von K. Rett et al ist es bekannt (Diabetes und Stoffwechsel, 1996, 5/3, Suppl. (59-63)), dass bei übergewichtigen Patienten Gaben von α-Liponsäure deren Beschwerden lindern. Weiter zeigt eine an Ratten durchgeführte Untersuchung von Min-Seon Kim et. al. (NATURE MEDCINE Band 10, Nr. 7, Juli 2004, Seiten 727-734), dass der α-Liponsäure eine gewisse appetitunterdrückende Wirkung zugeschrieben werden kann. Für den Menschen zeichnet sich daher die Möglichkeit ab, dass ein durch Aufnahme von α-Liponsäure vermindertes Bedürfnis zur Nahrungsaufnahme zu einer Gewichtsreduzierung führt.

Dementsprechend ist es Aufgabe der Erfindung, eine nebenwirkungslose Zusammensetzung der eingangs genannten Art anzugeben, die zur Gewichtsreduzierung beitragen oder diese verbessern kann.

Aus dem Dokument WO 03/007907 ist ein Ubichinon-Konzentrat bekannt, welches aus einem Emulgator, dem Ubichinon Q₁₀ sowie einem leichten pflanzlichen Öl (Distelöl) besteht. Dieses Konzentrat hat die Eigenschaft, das Q₁₀, das in den Mitochondrien zum Fettabbau benötigt wird, dem Organismus leichter verfügbar zu machen.

In US 6,300,377 B1 werden Zusammensetzungen aus mindestens fünf Komponenten beschrieben, welche Q₁₀, Polysorbat als Emulgator, Pflanzenöl oder Triglycerid, zumindest einen weiteren Glycerinester und beispielsweise α-Liponsäure umfassen.

Die deutsche Offenlegungsschrift DE 101 08 614 A1, Ausführungsbeispiel d, lehrt die Herstellung eines aus α-Liponsäure und Polysorbat bestehenden, wasserlöslichen α-Liponsäure-Solubilisats.

Dazu schlägt die Erfindung ein wasserfreies Konzentrat vor, welches aus demdas Ubichinon Q₁₀, einem mittelkettigens Triglycerid oder einem Triglyceridgemisch, α-Liponsäure und/oder deren Derivaten sowie einemn oder mehreren nichtionischen Polysorbaten, insbesondere Polysorbat 20 und/oder Polysorbat 80, als Emulgatoren mit einem HLB-Wert zwischen 9 und 19 besteht. Ebenso schlägt die Erfindung ein wasserfreies Konzentrat vor, welches Ubichinon Q₁₀, ein mittelkettiges Triglycerid oder Triglyceridgemisch, α-Liponsäure und/oder deren Derivate sowie einen oder mehrere Emulgatoren mit einem HLB-Wert von 9 bis 19, welcher aus Polysorbaten, insbesondere Polysorbat 20 und/oder Polysorbat 80, besteht, enthält, bei welchem das Verhältnis des Gewichtsanteils des Polysorbats zu der Summe der Gewichtsanteile der übrigen Bestandteile 4 : 1 bis 5, 5 : 1 beträgt.

Die Erfindung basiert auf der Konzeption, einerseits den Fettabbau durch Bereitstellung einer ausreichenden Menge an Q₁₀ zu fördern und andererseits den Fettabbau auf im Organismus gespeichertes Fett zu beschränken, indem die gleichzeitige Gabe von α-Liponsäure eine Nahrungsneuaufnahme durch Beeinflussung des Hypothalamus bremst. Diese genannten Bestandteile des erfindungsgemäßen Konzentrats sind lebensmittelrechtlich zugelassen und nebenwirkungsfrei. Das Konzentrat ist bei geeigneten Gewichtsanteilen seiner Bestandteile bei Raumtemperatur klar und viskos und lässt sich vorzugsweise bei leicht erhöhter Temperatur von etwa 60 °C ohne Schwierigkeiten zu einem Inhalt von Kapseln verarbeiten. Die tägliche Verabreichung einer derartigen Kapsel kann eine Gewichtsabnahme des Organismus bewirken. So hat eine inzwischen durchgeführte wissenschaftliche Untersuchung ergeben, dass Gaben des erfindungsgemäßen Konzentrats im Vergleich zu Placebo bei den Probanden zu einem stärkeren prozentualen Gewichtsverlust, das heißt zu einem größeren prozentualen visceralen Fettmasseverlust und einer größeren prozentualen Differenz im Taillenumfang führte.

Die für die Erfindung verwendbaren Emulgatoren sind durch die jeweiligen nationalen oder internationalen lebensmittel- oder arzneimittelrechtlichen Zulassungen bestimmt. Die in diesem Sinne zuvörderst in Betracht kommenden Lösungsvermittler sind die überall zugelassenen nichtionischen Polysorbate, allen voran Polysorbat 20 und/oder Polysorbat 80. Beispielsweise sind in den Vereinigten Staaten von Amerika und in Japan noch weitere Emulgatoren im Lebensmittelbereich zugelassen, die für die Erfindung ebenfalls eingesetzt werden kommen.

Ergänzend zur der α-Liponsäure kann auch die Dihydroliponsäure oder Dihydroliponamid mit Erfolg für das erfindungsgemäße Konzentrat eingesetzt werden. Im übrigen sind bevorzugte Ausführungsformen der Erfindung in den Unteransprüchen angegeben. So erweist es sich als zweckmäßig, wenn das Konzentrat entweder nur Polysorbat 80 oder gegebenenfalls eine Mischung aus Polysorbat 80 mit Polysorbat 20 enthält. Weiter empfiehlt es sich, als mittelkettiges Triglycerid entweder ein leichtes Nahrungsmittelöl eine Zusammensetzung zu verwenden, die im wesentlichen aus der Caprylsäure und der Caprinsäure besteht und unter Handelsnamen Miglyol 812 erhältlich ist.

Bevorzugt beträgt das Verhältnis des Gewichtsanteils des Polysorbats zu der Summe der Gewichtsanteile der übrigen Bestandteile des erfindungsgemäßen Konzentrats etwa 4 : 1 bis etwa 5,5 : 1. Das Gewichtsverhältnis von Q₁₀ zu α-Liponsäure liegt zweckmäßig etwa zwischen 1 : 1 bis etwa 1 : 4 mit bis zu 20%iger Abweichung.

Spezielle beispielhafte Zusammensetzungen des erfindungsgemäßen Konzentrats sind in den Unteransprüchen angegeben.

Das erfindungsgemäße Konzentrat eignet als Zusatz zu nicht-alkoholischen Getränken wie etwa Wasser, Fruchtsaft, Gemüsesaft, wobei sich eine Konzentration von Konzentrat zu Getränk von etwa 1 : 0,1 bis etwa 1 : 5000 empfiehlt. Auch Milchprodukten, Honig, Pflanzenölen kann das Konzentrat zugesetzt werden, wobei ein Verhältnis von Konzentrat zu zu den letztgenannten Produkten von 1 : 0,1 bis etwa 1 : 100 zweckmäßig ist.

Zur Herstellung des erfindungsgemäßen Konzentrats geht man zweckmäßig so vor, dass zunächst ein Solubilisat aus Q₁₀, Polysorbat 80 und einem mittelkettigen Triglycerid, sodann ein Solubilisat aus α-Liponsäure und Polysorbat 80 oder Polysorbat 20 gewonnen wird und anschließend das Q₁₀-Solubilisat mit dem α-Liponsäure-Solubilisat gemischt und zu einer homogenen, klaren und in Wasser löslichen Masse verrührt wird. Es empfiehlt sich, das Q₁₀-Solubilisat mit dem α-Liponsäure-Solubilisat im Gewichtsverhältnis von etwa 2:1, beispielsweise 1,8 : 1 zweckmäßig bei einer Temperatur von etwa 60°C zu mischen. Die optimale Solubilisierungstemperatur für α-Liponsäure liegt wesentlich höher als diejenige für das wärmeempfindliche Q₁₀, so dass sich die separate sedimentfreie Solubilisierung für die beiden Wirkstoffe bei den für diese jeweils geeigneten Temperaturen empfiehlt.

Ausführungsbeispiele der Erfindung wird nachstehend im Einzelnen erläutert und angegeben.

Ausgegangen wird von einem 5%igen wasserfreien, wasserlöslichen Q₁₀-Solubilisat, wie es in dem Herstellungsbeispiel 2 in dem Dokument WO 03/007907 beschrieben ist. Danach werden 790 Gewichtsteile Polysorbat 80 auf ca. 85 °C erhitzt. Dann werden 50 Gewichtsteile Coenzym Q₁₀ hinzugegeben und die Mischung (840 Gewichtsteile) unter Beibehaltung der Temperatur von ca. 85 °C so lange (etwa 5 Minuten) gerührt, bis sie homogen und transparent geworden ist. Anschließend werden dieser Mischung 160 Gewichtsteile Distelöl zugegeben, nachdem dieses zuvor ebenfalls auf ca. 85 °C erwärmt worden war, und unter Beibehaltung der Temperatur von ca. 85 °C so lange (etwa 2 Minuten) gerührt, bis die gesamte Mischung (1000 Gewichtsteile) ebenfalls homogen und transparent geworden ist. Nach Abkühlung auf Raum- oder Körpertemperatur bleiben Klarheit und Wasserlöslichkeit erhalten. Ein Gramm dieses Solubilisats enthält 50 mg Q₁₀.

Dort ist zwar als Bestandteil Distelöl angegeben. Erfindungsgemäß kann das Distelöl durch eine mittelkettige Triglyceridmischung gleicher Menge ersetzt werden, das gesättigte Pflanzenfettsäuren mittlerer Kettenlänge enthält, im Wesentlichen aus Caprylsäure und Caprinsäure besteht und beispielsweise von der Firma Sasol GmbH unter dem Namen Miglyol 812 N angeboten wird.

Sodann wird ein 10%iges wasserfreies, wasserlösliches α-Liponsäure-Solubilisat hergestellt, indem 900 Gewichtsteile Polysorbat 20 zunächst auf etwa 60 °C erwärmt werden. In das warme Polysorbat 20 werden langsam 100 Gewichtsteile α-Liponsäure (CAS-Nr. 62-46-4; ALIPURE der Firma Degussa) eingerieselt. Unter fortgesetztem Rühren wird das Gemisch so lange thermisch behandelt, bis dieses bei ca. 100 °C zu einer transparenten Mischung wird. Nach Abkühlen auf Raumtemperatur bleibt die Mischung transparent und ist in dieser Form vollständig wasserlöslich. 1 g dieses Solubilisats enthält 100 mg α-Liponsäure. Die Benutzung von Polysorbat 20 erleichtert zwar die Solubilisierung; jedoch ist an seiner Stelle eine gleiche Menge von Polysorbat 80 aus sensorischen Gründen vorzuziehen.

Zur Gewinnung eines Q₁₀-α-Liponsäure-Solubilisates werden etwa 660 Gewichtsteile des Q₁₀-Solubilisates mit etwa 370 Gewichtsteilen des α-Liponsäure-Solubilisates bei einer Temperatur von etwa 60 °C zu einer homogenen Mischung verrührt. Diese Mischung enthält 33 Gewichtsteile Q₁₀ und 37 Gewichtsteile α-Liponsäure, die beide in Polysorbat-Micellen von etwa 10 nm Teilchendurchmesser eingeschlossen sind. Mit dieser Mischung wird eine Kapsel aus Gelatine oder gelatinefrei mit 470 mg Füllgewicht befüllt. Der Inhalt dieser Kapsel besteht dann aus etwa 15,02 mg Q₁₀, etwa 16,68 mg α-Liponsäure, etwa 48,22 mg Triglyceriden und etwa 389,7 mg Polysorbat 80.

Bei einer Aufnahme von beispielsweise drei derartiger Kapseln pro Tag werden daher vom Organismus aufgenommen etwa
- 45,15 mg Q₁₀
- 50,58 mg α-Liponsäure
- 144,66 mg Triglyceride
- 1169,1 mg Polysorbat 80.

Diese Mengen bleiben weit unterhalb derjenigen maximalen Tagesdosis, die amtlicherseits für die jeweiligen Bestandteile lebensmittelrechtlich zugelassen ist.

Weitere Beispiele für die Zusammensetzung des erfindungsgemäßen Konzentrats sind in den nachstehenden Tabellen angegeben. In diesen bezieht sich MCT auf das oben erwähnte Miglyol 812 und unter Polysorbat ist das Polysorbat 80 zu verstehen. Die Herstellung der einzelnen Konzentratbeispiele folgt der oben für das erste Beispiel gegebenen Erläuterung.

### Beispiel 2

| | g/kg | Gew% |
|---|---|---|
| A) Q₁₀ | 50 | 5 |
| B) α-Liponsäure | 100 | 10 |
| C) MCT | 40 | 4 |
| D) Polysorbat | 810 | 81 |
| Summen: | 1.000 | 100 |

### Beispiel 3

| | g/kg | Gew% |
|---|---|---|
| A) Q₁₀ | 40 | 4 |
| B) α-Liponsäure | 80 | 8 |
| C) MCT | 60 | 6 |
| D) Polysorbat | 820 | 82 |
| Summen: | 1.000 | 100 |

### Beispiel 4

| | g/kg | Gew% |
|---|---|---|
| A) Q₁₀ | 50 | 5 |
| B) α-Liponsäure | 90 | 9 |
| C) MCT | 50 | 5 |
| D) Polysorbat | 810 | 81 |
| Summen: | 1.000 | 100 |

### Beispiel 5

| | g/kg | Gew% |
|---|---|---|
| A) Q₁₀ | 20 | 2 |
| B) α-Liponsäure | 80 | 8 |
| C) MCT | 60 | 6 |
| D) Polysorbat | 840 | 84 |
| Summen: | 1.000 | 100 |

Das erfindungsgemäße Q₁₀-α-Liponsäure-Konzentrat kann aufgrund seiner Wasserlöslichkeit, insbesondere in leicht erwärmtem (etwa 35 °C) Wasser, alkoholfreien Getränken in geeigneter Dosis beigemischt werden, ohne dass die Klarheit des Getränks Einbußen erleidet. Ferner kann das erfindungsgemäße Konzentrat Salben oder anderen kosmetischen Mitteln beigegeben werden, weil die Micellenstruktur des Konzentrats eine Penetration in die Haut erleichtert. Schließlich bietet sich das erfindungsgemäße Konzentrat als Nahrungsergärtzungsmittel oder, in höherer Dosierung, auch als dietätisches Lebensmittel an.

## Patentansprüche

1. Wasserfreies Konzentrat, enthaltend Ubichinon Q₁₀, ein mittelkettiges Triglycerid oder Triglyceridgemisch, α-Liponsäure und/oder deren Derivate sowie einen oder mehrere Emulgatoren mit einem HLB-Wert von 9 bis 19, welcher aus Polysorbaten, insbesondere Polysorbat 20 und/oder Polysorbat 80, besteht, bei welchem das Verhältnis des Gewichtsanteils des Polysorbats zu der Summe der Gewichtsanteile der übrigen Bestandteile 4 : 1 bis 5,5 : 1 beträgt.

2. Wasserfreies Konzentrat, bestehend aus Ubichinon Q₁₀, einem mittelkettigen Triglycerid oder Triglyceridgemisch, α-Liponsäure und/oder deren Derivaten sowie einem oder mehreren nichtionischen Polysorbaten, insbesondere Polysorbat 20 und/oder Polysorbat 80, als Emulgatoren mit einem HLB-Wert von 9 bis 19.

3. Konzentrat nach Anspruch 1 oder 2, bei dem der Emulgator aus Polysorbat 20 und/oder Polysorbat 80 besteht.

4. Konzentrat nach einem der Ansprüche 1 bis 3, bei dem das α-Liponsäurederivat Dihydroliponsäure oder Dihydroliponamid ist.

5. Konzentrat nach einem der vorstehenden Ansprüche, bei welchem das mittelkettige Triglycerid ein leichtes Öl oder eine Mischung aus Caprylsäure und Caprinsäure ist.

6. Konzentrat nach einem der Ansprüche 2 bis 5, bei welchem das Verhältnis des Gewichtsanteils des Polysorbats zu der Summe der Gewichtsanteile der übrigen Bestandteile etwa 4 : 1 bis etwa 5,5 : 1 beträgt.

7. Konzentrat nach einem der vorstehenden Ansprüche, bei welchem das Gewichtsverhältnis des Ubichinons zu α-Liponsäure etwa zwischen 1 : 1 und etwa 1 : 4 liegt:

8. Konzentrat nach einem der vorstehenden Ansprüche, welches aus etwa 85 Gew% Polysorbaten, etwa 3,3 Gew% Q₁₀, etwa 4 Gew% α-Liponsäure und etwa 10 Gew% Triglyceriden besteht.

9. Konzentrat nach einem der Ansprüche 1 bis 7, welches aus etwa 5 Gew% Q₁₀, etwa 10 Gew% α-Liponsäure, etwa 4 Gew% Triglyceriden und etwa 81 Gew% Polysorbat 80 besteht.

10. Konzentrat nach einem der Ansprüche 1 bis 7, welches aus etwa 4 Gew% Q₁₀, etwa 8 Gew% α-Liponsäure, etwa 6 Gew% Triglyceriden und etwa 82 Gew% Polysorbat 80 besteht.

11. Konzentrat nach einem der Ansprüche 1 bis 7, welches aus etwa 5 Gew% Q₁₀, etwa 9 Gew% α-Liponsäure, etwa 5 Gew% Triglyceriden und etwa 81 Gew% Polysorbat 80 besteht.

12. Konzentrat nach einem der Ansprüche 1 bis 7, welches aus etwa 2 Gew% Q₁₀, etwa 8 Gew% α-Liponsäure, etwa 6 Gew% Triglyceriden und etwa 84 Gew% Polysorbat 80 besteht.

13. Verfahren zur Herstellung eines Konzentrats nach einem der vorstehenden Ansprüche, bei welchem zunächst ein Solubilisat aus Q₁₀, Polysorbat 80 und einem Triglycerid, sodann ein Solubilisat aus α-Liponsäure und Polysorbat 80 oder Polysorbat 20 gewonnen wird und anschließend das Q₁₀-Solubilisat mit dem α-Liponsäure-Solubilisat gemischt und zu einer homogenen Masse verrührt wird, wobei das α-Liponsäure-Solubilisat bei einer höheren Temperatur von beispielsweise 100°C gewonnen wird als diejenige Temperatur,von beispielsweise 85°C, bei welcher das Q₁₀ solubiliert wird, und beide Solubilisate bei niedrigerer Temperatur von beispielsweise 60°C vermischt werden.

14. Verfahren nach Anspruch 13 , bei welchem etwa 2, zweckmäßig etwa 1,8 Teile des Q₁₀ Solubilisats mit etwa 1 Teil des α-Liponsäure-Solubilisats vermischt werden.

15. Konzentrat nach einem der Ansprüche 1 bis 12, welches einem nicht-alkoholischen Getränk im Verhältnis ein Teil Konzentrat zu etwa 0,1 bis etwa 5000 Teilen Getränk zugesetzt ist.

16. Konzentrat nach einem der Ansprüche 1 bis 12, welches einem Milchprodukt, Honig, Pflanzenöl oder dergleichen Lebensmitteln im Verhältnis ein Teil Konzentrat zu etwa 0,1 bis etwa 500 Teilen Lebensmittel zugesetzt ist.

## Claims

1. Water-free concentrate containing ubichinon Q₁₀, a medium-chained triglyceride or triglyceride mixture, α-lipoic acid and/or derivatives thereof as well as one or more emulsifiers having an HLB value of 9 to 19, which emulsifier consists of polysorbates, in particular polysorbate 20 and/or polysorbate 80, wherein the ratio of the weight component of the polysorbate to the total of the weight components of the remaining ingredients is 4:1 to 5.5:1.

2. Water-free concentrate consisting of ubichinon Q₁₀, a medium-chained triglyceride or triglyceride mixture, α-lipoic acid and/or derivatives thereof as well as one or more non-ionic polysorbates, in particular polysorbate 20 and/or polysorbate 80, as emulsifiers having an HLB value of 9 to 19.

3. Concentrate as claimed in Claim 1 or 2, wherein the emulsifier consists of polysorbate 20 and/or polysorbate 80.

4. Concentrate as claimed in any one of Claims 1 to 3, wherein the α-lipoic acid derivative is dihydrolipoic acid or dihydrolipoamide.

5. Concentrate as claimed in any one of the preceding Claims, wherein the medium-chained triglyceride is a light oil or a mixture of caprylic acid and caprinic acid.

6. Concentrate as claimed in any one of Claims 2 to 5, wherein the ratio of the weight component of the polysorbate to the total of the weight components of the remaining ingredients is approximately 4:1 to approximately 5.5:1.

7. Concentrate as claimed in any one of the preceding Claims, wherein the weight ratio of the ubichinon to α-lipoic acid is approximately between 1:1 and approximately 1:4.

8. Concentrate as claimed in any one of the preceding Claims, which consists of approximately 85 wt.% polysorbates, approximately 3.3 wt.% Q₁₀, approximately 4 wt.% α-lipoic acid and approximately 10 wt.% triglycerides.

9. Concentrate as claimed in any one of Claims 1 to 7, which consists of approximately 5 wt.% Q₁₀, approximately 10 wt.% α-lipoic acid, approximately 4 wt.% triglycerides and approximately 81 wt.% polysorbate 80.

10. Concentrate as claimed in any one of Claims 1 to 7, which consists of approximately 4 wt.% Q₁₀, approximately 8 wt.% α-lipoic acid, approximately 6 wt.% triglycerides and approximately 82 wt.% polysorbate 80.

11. Concentrate as claimed in any one of Claims 1 to 7, which consists of approximately 5 wt.% Q₁₀, approximately 9 wt.% α-lipoic acid, approximately 5 wt.% triglycerides and approximately 81 wt.% polysorbate 80.

12. Concentrate as claimed in any one of Claims 1 to 7, which consists of approximately 2 wt.% Q₁₀, approximately 8 wt.% α-lipoic acid, approximately 6 wt.% triglycerides and approximately 84 wt.% polysorbate 80.

13. Method of producing a concentrate as claimed in any one of the preceding Claims, in which firstly a solubilisate is obtained from Q₁₀, polysorbate 80 and a triglyceride, and then a solubilisate is obtained from α-lipoic acid and polysorbate 80 or polysorbate 20, and subsequently the Q₁₀ solubilisate is mixed with the α-lipoic acid solubilisate and stirred to form a homogenous mass, wherein the α-lipoic acid solubilisate is obtained at a higher temperature of e.g., 100°C than the temperature of e.g., 85°C at which the Q₁₀ solubilises, and the two solubilisates are mixed at a lower temperature of e.g., 60°C.

14. Method as claimed in Claim 13, wherein approximately 2, in an expedient manner approximately 1.8, parts Q₁₀ solubilisate is mixed with approximately 1 part α-lipoic acid solubilisate.

15. Concentrate as claimed in any one of Claims 1 to 12, which is added to a non-alcoholic drink in the ratio of one part concentrate to approximately 0.1 to approximately 5000 parts drink.

16. Concentrate as claimed in any one of Claims 1 to 12, which is added to a dairy product, honey, vegetable oil or a similar foodstuff in the ratio of one part concentrate to approximately 0.1 to approximately 500 parts foodstuff.

## Revendications

1. Concentré anhydre, contenant de l'ubiquinone Q₁₀, un triglycéride de moyenne longueur de chaîne ou un mélange de tels triglycérides, de l'acide α-liponique et/ou ses dérivés ainsi qu'un ou plusieurs émulsifiants ayant une valeur d'équilibre hydrophile-lipophile de 9 à 19, qui est constitué de polysorbates, en particulier de Polysorbate 20 et/ou de Polysorbate 80, dans lequel le rapport de la fraction en poids du polysorbate à la somme des fractions en poids des autres composants s'élève à une valeur de 4 : 1 à 5,5 : 1.

2. Concentré anhydre, constitué d'ubiquinone Q₁₀, d'un triglycéride de moyenne longueur de chaîne ou d'un mélange de tels triglycérides, d'acide α-liponique et/ou de ses dérivés ainsi que d'un ou plusieurs polysorbates non ioniques, en particulier de Polysorbate 20 et/ou de Polysorbate 80, comme émulsifiants ayant une valeur d'équilibre hydrophile-lipophile de 9 à 19.

3. Concentré suivant la revendication 1 ou 2, dans lequel l'émulsifiant est constitué de Polysorbate 20 et/ou de Polysorbate 80.

4. Concentré suivant l'une des revendications 1 à 3, dans lequel le dérivé d'acide α-liponique est l'acide dihydroliponique ou le dihydroliponamide.

5. Concentré suivant l'une des revendications précédentes, dans lequel le triglycéride de moyenne longueur de chaîne est une huile légère ou un mélange d'acide caprylique et d'acide caprique.

6. Concentré suivant l'une des revendications 2 à 5, dans lequel le rapport de la fraction en poids du polysorbate à la somme des fractions en poids des autres constituants s'élève à une valeur d'environ 4 : 1 à environ 5,5 : 1.

7. Concentré suivant l'une des revendications précédentes, dans lequel le rapport en poids de l'ubiquinone à l'acide α-liponique se situe approximativement entre 1 : 1 et environ 1 : 4.

8. Concentré suivant l'une des revendications précédentes, qui est constitué d'environ 85 % en poids de polysorbates, d'environ 3,3 % en poids de Q₁₀, d'environ 4 % en poids d'acide α-liponique et d'environ 10 % en poids de triglycérides.

9. Concentré suivant l'une des revendications 1 à 7, qui est constitué d'environ 5 % en poids de Q₁₀, d'environ 10 % en poids d'acide α-liponique, d'environ 4 % en poids de triglycérides et d'environ 81 % en poids de Polysorbate 80.

10. Concentré suivant l'une des revendications 1 à 7, qui est constitué d'environ 4 % en poids de Q₁₀, d'environ 8 % en poids d'acide α-liponique, d'environ 6 % en poids de triglycérides et d'environ 82 % en poids de Polysorbate 80.

11. Concentré suivant l'une des revendications 1 à 7, qui est constitué d'environ 5 % en poids de Q₁₀, d'environ 9 % en poids d'acide α-liponique, d'environ 5 % en poids de triglycérides et d'environ 81 % en poids de Polysorbate 80.

12. Concentré suivant l'une des revendications 1 à 7, qui est constitué d'environ 2 % en poids de Q₁₀, d'environ 8 % en poids d'acide α-liponique, d'environ 6 % en poids de triglycérides et d'environ 84 % en poids de Polysorbate 80.

13. Procédé de production d'un concentré suivant l'une des revendications précédentes, dans lequel on prépare tout d'abord un solubilisat de Q₁₀, de Polysorbate 80 et d'un triglycéride, on prépare ensuite un solubilisat d'acide α-liponique et de Polysorbate 80 ou de Polysorbate 20, puis on mélange le solubilisat de Q₁₀ avec le solubilisat d'acide α-liponique et on agite le mélange pour former une masse homogène, le solubilisat d'acide α-liponique étant préparé à une température supérieure, par exemple 100°C, à la température, par exemple de 85°C, à laquelle Q₁₀ se solubilise, et on réunit les deux solubilisats en les mélangeant à une température plus basse, par exemple à une température de 60°C.

14. Procédé suivant la revendication 13, dans lequel environ 2 parties, mieux encore environ 1,8 partie du solubilisat de Q₁₀ sont mélangées avec environ 1 partie du solubilisat d'acide α-liponique.

15. Concentré suivant l'une des revendications 1 à 12, qui est ajouté à une boisson non alcoolisée dans le rapport d'une partie de concentré pour environ 0,1 à environ 5000 parties de boisson.

16. Concentré suivant l'une des revendications 1 à 12, qui est ajouté à un produit laitier, du miel, une huile végétale ou des produits alimentaires similaires dans le rapport d'une partie de concentré pour environ 0,1 à 500 parties de produit alimentaire.
